(19) Europäisches Patentamt
European Patent Office
Office européen des brevets

(11) Publication number: 0 024 105
A1

(12) EUROPEAN PATENT APPLICATION

(21) Application number: 80302206.0

(22) Date of filing: 01.07.80

(51) Int. Cl.$^3$: C 07 D 249/12
A 61 K 31/41

(30) Priority: 12.07.79 GB 7924235

(43) Date of publication of application:
25.02.81 Bulletin 81/8

(84) Designated Contracting States:
AT BE CH DE FR GB IT LI NL SE

(71) Applicant: BEECHAM GROUP LIMITED
Beecham House Great West Road
Brentford, Middlesex(GB)

(72) Inventor: Cassidy, Frederick
12 Peterswood
Harlow Essex(GB)

(72) Inventor: Moore, Richard William
34 Sunningdale Bishop
Stortford Hertfordshire(GB)

(74) Representative: Dawson, Hugh Bainforde, Dr. et al,
European Patent Attorney BEECHAM
PHARMACEUTICALS Yew Tree Bottom Road
Epsom, Surrey KT18 5XQ.(GB)

# (54) Prostaglandin analogons, their preparation and their pharmaceutical compositions.

(57) Compounds of the formula (I):

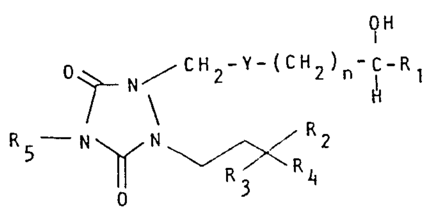

wherein:
n is 3 to 5;
Y is $-CH_2-CH_2$ or -CH=CH- or -C≡C-
$R_1$ is $C_{1-4}$ alkyl;
$R_2$ is hydrogen, $C_{1-4}$ alkyl or phenyl;
$R_3$ is hydroxy or protected hydroxy;
$R_4$ is hydrogen, $C_{1-9}$ alkyl, $C_{3-8}$ cycloalkyl, phenyl, naphthyl, any of which phenyl moieties ornaphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; and
$R_5$ is $C_{1-6}$ alkyl having useful pharmacological activity, compositions containing them and processes for their preparation.

EP 0 024 105 A1

see front page



# NOVEL ORGANIC COMPOUNDS

This invention relates to novel compounds having pharmacological activity, to a process for their preparation, to intermediates useful in that process and to pharmaceutical compositions containing them.

Offenlegungsschrift No. 2323193 discloses that pyrazolidine derivatives of the formula (I)' :

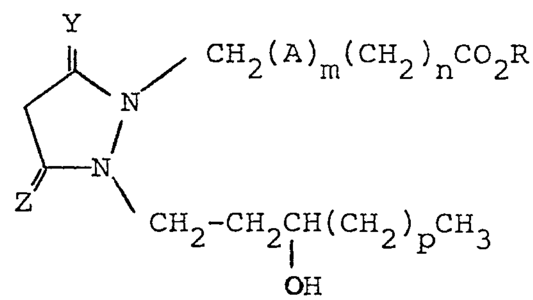

(I)'

wherein :

A is CH=CH or C=C; R is H, an alkali metal, an amine salt, or a $\not>$ 12C hydrocarbon or chlorohydrocarbon residue; m is 0 or 1; n is 0-6; p is 0-6; and Y and Z are O or H except that Y and Z are not both O; have similar biological properties to the prostaglandins or are antagonists of prostaglandins.

French Patent Application No. 2258376 discloses that 10-aza prostaglandins of formula (II)" :

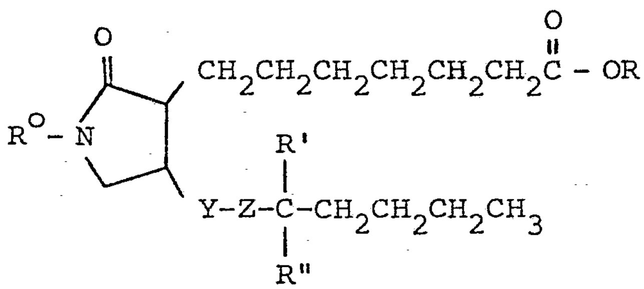

(II)"

wherein :

R=H or lower alkyl; R' and R" = $CH_3$ or $C_2H_5$; $R^O$=H or lower alkyl; Y= $-CH_2-CH_2-$, or -CH = CH-; Z = -CO or -CH(~OH)-; are useful in the treatment of blood pressure and gastro-intestinal disorders, and in the preparation for confinement.

Belgian Patent No.835989 discloses that compounds of the formula (III)" :

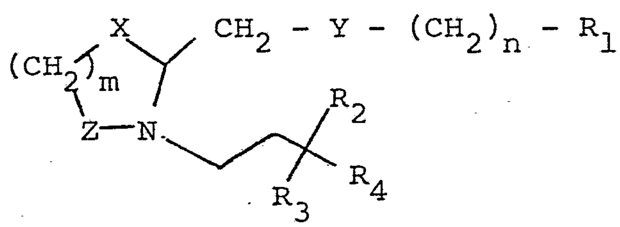

(III)"

wherein :

X is CO, protected CO, CROH in which R is hydrogen or $C_{1-4}$ alkyl and in which the OH moiety may be protected; Y is $CH_2CH_2$ or CH=CH; Z is CO or $CH_2$; n is 1 to 8; m is 1, 2 or 3; $R_1$ is hydrogen, $CH_2OH$, $CH_2OH$ in which the OH moiety is protected, $CO_2W$ wherein W is hydrogen or $CO_2W$ represents an ester group in which the ester moiety contains from 1 to 12 carbon atoms, or $CONH_2$; $R_2$ is hydrogen, $C_{1-4}$ alkyl, or taken together with $R_3$

and the carbon atom to which it is attached represents a carbonyl group; $R_3$ is hydrogen, hydroxy or protected hydroxy; $R_4$ is hydrogen or $C_{1-9}$ alkyl; and salts thereof; have useful pharmacological activity.

A novel class of compounds also having useful pharmacological activity has now been discovered, which compounds are structurally distinct from the prior art referred to above.

Accordingly the present invention provides a compound of the formula (I) :

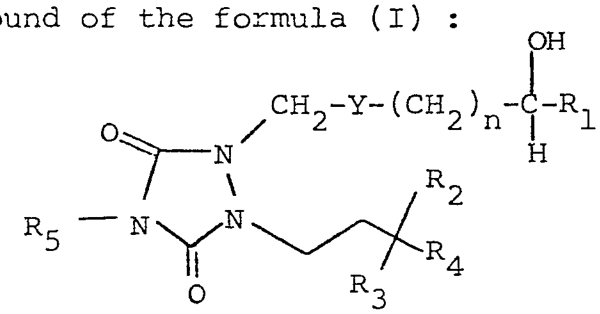

(I)

wherein :

n is 3 to 5;

Y is $-CH_2-CH_2$ or $-CH=CH-$ or $-C\equiv C-$

$R_1$ is $C_{1-4}$ alkyl;

$R_2$ is hydrogen, $C_{1-4}$ alkyl or phenyl;

$R_3$ is hydroxy or protected hydroxy;

$R_4$ is hydrogen, $C_{1-9}$ alkyl, $C_{3-8}$ cycloalkyl, phenyl, naphthyl, any of which phenyl moieties or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; and

$R_5$ is $C_{1-6}$ alkyl.

Preferably n is 4 or 5.

Suitable values for Y include $-CH_2-CH_2$ or <u>cis</u> $-CH=CH-$

A preferred value for Y is <u>cis</u> $-CH=CH-$

Suitable examples of $R_1$ include methyl, ethyl, <u>n</u>- and iso propyl, and <u>n</u>-, <u>sec</u>- and <u>tert</u>-butyl.

Suitable examples of $R_2$ include hydrogen, methyl, ethyl, and phenyl. Preferred examples of $R_2$ include methyl.

Suitable protected hydroxy groups $R_3$ include readily hydrolysable derivatives such as acylated hydroxy groups in which the acyl moiety contains 1 to 4 carbon atoms, for example the acetoxy group; and hydroxy groups etherified by inert groups such as benzyl or methyl. Preferably however $R_3$ is hydroxy.

Suitable groups $R_4$ when $R_4$ is an alkyl group include $C_{4-9}$ alkyl groups. Such $C_{4-9}$ alkyl groups may be straight chain alkyl groups, such as n-butyl, n-pentyl, n-hexyl and n-heptyl, or may be alkyl groups branched by one or two methyl groups (at the same or different carbon atoms). Thus for example, $R_4$ may be a group $CH_2R_6$, $CH(CH_3)R_6$ and $C(CH_3)_2R_6$ wherein $R_6$ is a straight chain alkyl group such that the carbon content of the resultant group $R_4$ is 4 to 9.

In general preferred groups $R_4$ when $R_4$ is an alkyl group include straight chain pentyl, hexyl and heptyl groups. Other preferred groups $R_4$ include groups $CH(CH_3)R_6$ $C(CH_3)_2R_6$ wherein $R_6$ is straight chain butyl, pentyl and hexyl.

When $R_4$ is a $C_{3-8}$ cycloalkyl moiety, the moiety may suitably be a $C_{5-8}$ cycloalkyl moiety such as a cyclohexyl moiety. It may also be a cyclopropyl moiety.

When $R_4$ is an aryl group as previously defined, suitable groups $R_4$ include phenyl and naphthyl which groups may be substituted by normally one, two or three groups selected from those substituent groups listed hereinbefore. Examples of suitable substituent groups include fluorine,

chlorine and bromine atoms and $CF_3$, methyl, ethyl, _n_- and _iso_-propyl, methoxy, ethoxy, _n_- and _iso_-propoxy and nitro groups. Other examples of such groups include hydroxy and benzyloxy. Preferably the aryl moieties when substituted by such groups will be mono- or disubstituted.

Suitable examples of $R_5$ include methyl, ethyl, _n_- and _iso_-propyl and _n_-butyl and such groups branched in the alkyl moiety by one or two methyl groups (at the same or different carbon atoms). Preferred $R_5$ groups include methyl.

One particularly suitable sub-group of compounds within formula (I) is of formula (II) :

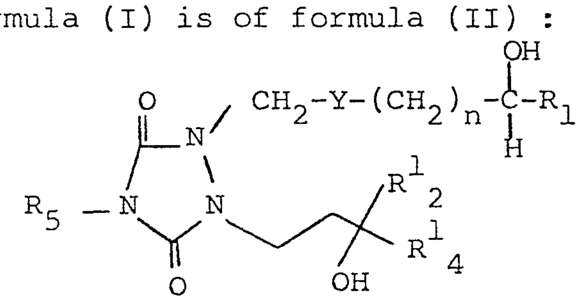

(II)

wherein :

Y, n, $R_1$ and $R_5$ are as defined in formula (I);
$R^1_2$ is hydrogen, methyl, ethyl or phenyl; and
$R^1_4$ is hydrogen or $C_{1-9}$ alkyl

Preferably n is 4 or 5.

Suitably Y is $-CH_2CH_2-$ or <u>cis</u> -CH=CH-
A preferred value for Y is <u>cis</u> -CH=CH-
Suitable $R_1$ are as listed as suitable under formula (I)
Suitable $R^1_2$ may be hydrogen, methyl or ethyl.

While $R^1_4$ may be hydrogen or a $C_{1-9}$ alkyl group in formula (II), it is normally a $C_{4-9}$ alkyl group. In such cases suitable and preferred straight chain and branched groups $R^1_4$ include those previously described as suitable and preferred for the group $R_4$ when $R^1_4$ is a $C_{4-9}$ alkyl group. Such preferred groups $R^1_4$

include straight chain pentyl, hexyl and heptyl. Other preferred groups $R^1_4$ include $CH(CH_3)R^1_6$ and $C(CH_3)_2R^1_6$ wherein $R^1_6$ is straight chain butyl, pentyl or hexyl.

Preferably $R_5$ is methyl or ethyl, in particular methyl.

From the aforesaid it will be realised that one preferred group within formula (II) is of formula (III) :

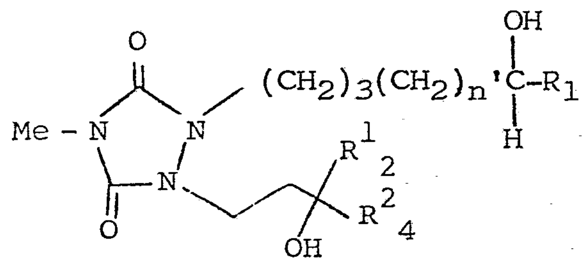

(III)

wherein:

n' is 4 or 5

$R_1$ is as defined in formula (I);

$R^1_2$ is hydrogen, methyl or ethyl; and

$R^2_4$ is a $C_{4-9}$ alkyl group.

Suitable $R_1$ are as listed as suitable under formula (I). Suitable and preferred groups $R^2_4$ include those listed hereinbefore for $R^1_4$ when $R^1_4$ is a $C_{4-9}$ alkyl group.

Another preferred group within formula (II) is of formula (IV):

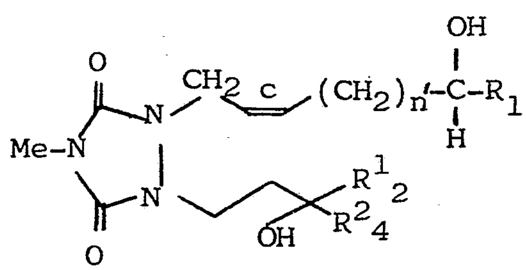

(IV)

wherein the variables are as defined in formula (III). Suitable and preferred values of $R_1$ and $R^1_4$ are as hereinbefore described for compounds of formula (III).

Another suitable sub-group of compounds within formula (I) is of formula (V):

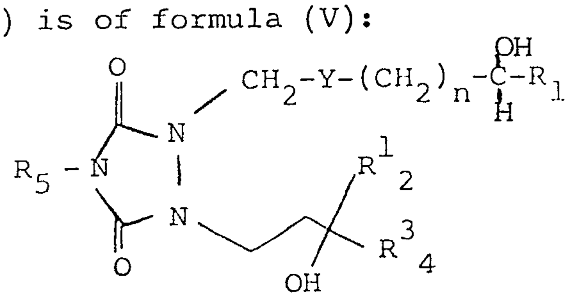

(V)

wherein :

Y, n, $R_1$, and $R_5$ are as defined in formula (I);

$R^1_2$ is hydrogen, methyl, ethyl or phenyl; and

$R^3_4$ is a group of formula (VI):

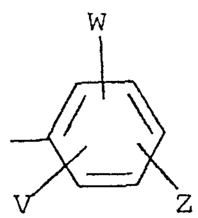

(VI)

wherein :

V, W and Z are each hydrogen or fluorine, chlorine or bromine atoms, or $CF_3$, methyl, ethyl, n- or iso-propyl, methoxy, ethoxy, n or iso-propoxy or nitro groups.

In formula (V) it is preferred that n is 4

Suitable $R_1$ are as listed as suitable under formula (I).

In formula (VI) V and W will often be hydrogen.

Often in formula (V) $R_5$ will be methyl or ethyl, preferably methyl.

A further sub-group of compounds within the formula (I) is of formula (VII):

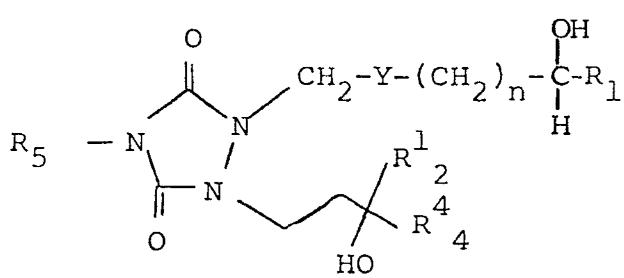

(VII)

wherein the variable groups are as defined in formula (II) and $R^4_4$ is a group of formula (VIII):

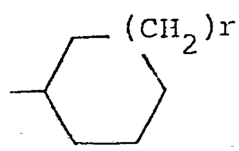

(VIII)

wherein r is 0-3, more suitably 1.

Suitable and preferred variable groups except for $R^4_4$ in formula (VIII) are as in formula (II).

A group of compounds within the formula (VIII) is of formula (IX):

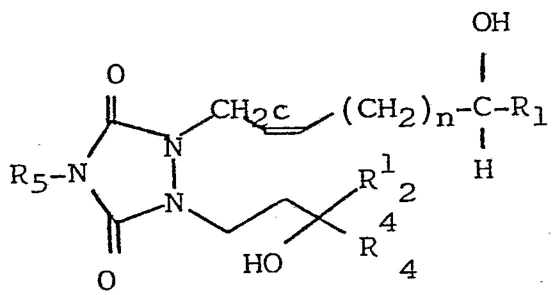

(IX)

wherein the variable groups are as defined in formula (VIII). Suitable and preferred values of $R_1$, $R^1_2$ and $R_5$ are as so described under formula (II).

Process Variant a)

The invention also provides a process for the preparation of a compound of the formula (I), which process comprises reacting a compound of formula (X):

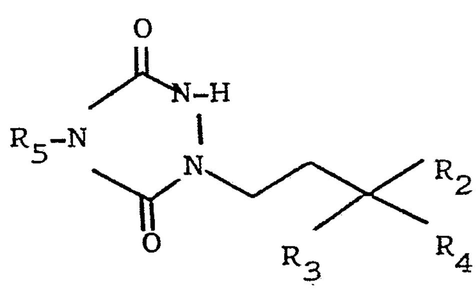

(X)

wherein $R_2$, $R_3$, $R_4$, and $R_5$ are as defined in formula (I) with a compound of formula (XI):

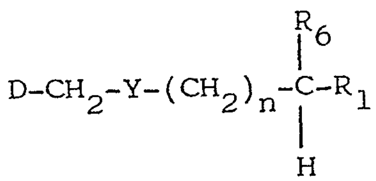

(XI)

wherein D is a group readily displaced by nucleophiles; $R_6$ is hydroxy or protected hydroxy; and Y, n and $R_1$ are as defined in formula (I), and thereafter deprotecting $R_6$ when protected hydroxy.

The reaction is suitably carried out in an inert solvent, such as hexamethylphosphoramide or N,N-dimethylformamide, as room temperature, in the presence of a base such as lithium carbonate.

Protected $R_6$ hydroxy groups may be converted to hydroxy $R_6$ groups by for example, de-acylation or de-alkylation reactions.

Suitable examples of D include activated ester moieties such as tosylate and mesylate and halogens such as bromide and iodide, preferably mesylate or bromide.

Suitable protected hydroxy groups $R_6$ are as hereinbefore described for protected hydroxy groups $R_3$.

Process Variant b)

The invention also provides a further process for the preparation of a compound of the formula (I) which process comprises reacting a compound of formula (XII):

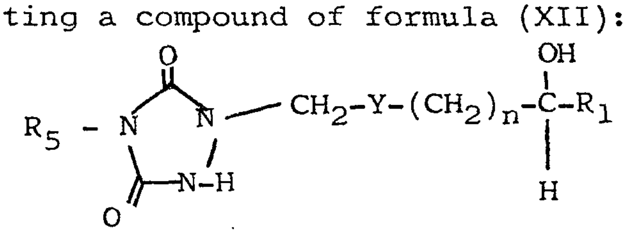

(XII)

wherein:

Y is $CH_2$-$CH_2$- or -CH=CH-; and

n, $R_1$ and $R_5$ are as defined in formula (I), with a compound of formula (XIII):

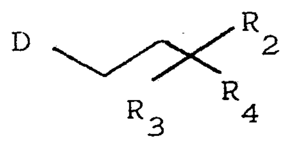

(XIII)

wherein D is a group readily displaced by nucleophiles, and $R_2$, $R_3$ and $R_4$ are as defined in formula (I).

This reaction is suitable carried out in an inert organic solvent, such as hexamethylphosphoramide or N,N-dimethylformamide, at room temperature, in the presence of a base, such as sodium carbonate or sodium hydride, and a source of halide ions, such as an alkali metal halide. Suitable alkali halides include sodium iodide and lithium iodide.

Suitable examples of D include those hereinbefore described for compounds of formula (XI). Preferably D is a tosylate residue.

Process Variant c)

The present invention also provides another process for the preparation of a compound of the formula (I) which process comprises reacting a compound of the formula (XIV)

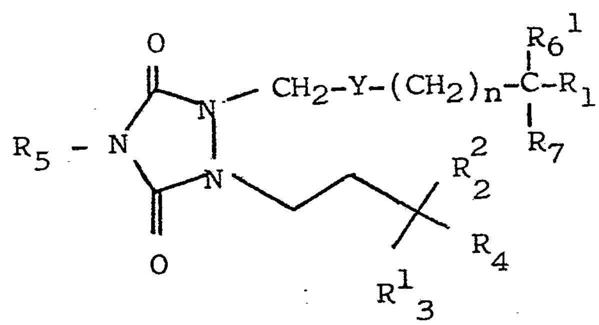

(XIV)

wherein $R^2_2$ and $R^1_3$ are as defined for $R_2$ and $R_3$ in formula (I) or $R^2_2$ and $R^1_3$ together form an oxo group; $R^1_6$ is hydroxy or protected hydroxy; $R_7$ is hydrogen or $R^1_6$ and $R_7$ together form an oxo group and at least one of $R^2_2 + R^1_3$ and $R^1_6 + R_7$ are oxo with either i) a carbonyl reducing agent or (when $R^1_6$ is hydroxy or protected hydroxy) ii) an alkyl or phenyl Grignard reagent (or similar metallic complex) and thereafter deprotecting $R^1_6$ when protected hydroxy.

The necessary reaction of a compound of the formula (XIV) may be carried out with any of the usual carbonyl-

usual carbonyl-reducing agents or Grignard reagents under conditions which will be readily apparent to the skilled man.

Suitably borohydrides, such as sodium or lithium borohydrides in a solvent such as diglyme, an alcohol or an ether, or $BH_3$ in tetrahydroguran may be used in the reduction step.

Protected $R^1_6$ hydroxy groups may be deprotected as described under process variant a).

The preparation of intermediates of the formulae (X), (XII) and (XIV) may be prepared by methods described in published European Patent Application No. 79301142.0 or by analogous methods thereto.

The groups $R_3$ and Y in the compounds of formula (I) may be varied by any conventional reactions. Thus for example protected hydroxy $R_3$ groups may be converted to hydroxy $R_3$ groups by de-acylation or de-alkylation.

Similarly, compounds of the formula (I) wherein Y is a -C≡C- or -CH=CH- group may be converted to their corresponding -CH=CH- or $-CH_2-CH_2-$ analogues by any of the usual reduction methods, for alkenes and alkynes such as palladium catalysed hydrogenation.

It will of course be realised that the compounds of the formula (I) have asymmetric centres, and thus are capable of existing in enantiomeric and diasteriomeric forms. The invention extends to each of these isomeric forms, and to mixtures thereof.

It will be realised that when Y is CH=CH, this group may exist in the E and Z conformations. The invention extends to compounds of the formula (I) containing Y in each of these isomeric forms.

The different isomeric forms may be resolved or separated as appropriate by the usual methods.

Compounds of the formula (I) have useful pharmacological activity. For example compounds

of the formula (I) have anti-gastric secretion activity; anti-ulcer activity; activity on smooth muscle, such as vascular activity, e.g. anti-hypertensive activity, effects on the respiratory tract, e.g. bronchodilation activity, anti-fertility activity and gastrointestinal smooth muscle activity; platelet aggregation inhibition acitivity and/ or cardiac activity, e.g. anti-arrhythmic acitivity and/or anti-hypertensive activity.

Compounds of the formula (I) may accordingly be used in the treatment of the corresponding disorders in humans and animals.

The compounds of the fomula (I) are especially useful bronchodilation agents.

In general it may be said that compounds of the formula (I) have a range of pharmacological activities similar to those shown by the natural prostaglandins, but that their activity profiles tend to be rather more selective, so that each compound tends to have a major activity readily ascertained by routine pharmacological tests.

The invention therefore also provides a pharmaceutical composition comprising a compound of the formula (I) and a pharmaceutically acceptable carrier.

In order to utilise the selectivity of activity found with compounds of the formula (I), normally a given compound will be used in the treatment of the disorder corresponding to the compound's major activity (that is, the disorder for which the compound has the lowest active dose) and will accordingly be formulated into the corresponding pharmaceutical composition, and administered in a manner conventional for treatment of that disorder. It may also of course be possible with compounds having one or more further pronounced activities to formulate and use the compound for those

further activities as well as for the major activity, provided that there is no undesirable pharmacological interaction between the different activities, or that separation of the different activities can be obtained by a difference in the formulation or in the mode of administration.

The compositions may be in the form of tablets, capsules, powders, granules, lozenges or liquid preparations, such as oral or sterile parenteral solutions or suspensions.

Tablets and capsules for oral administration may contain conventional excipients such as binding agents, fillers, tabletting lubricants, disintegrants and acceptable wetting agents and the like. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives such as suspending agents, emulsifying agents, nonaqueous vehicles (which may include edible oils), preservatives, and if desired conventional flavouring or colouring agents, and the like.

For parenteral administration, fluid unit dosage forms may be prepared utilizing the compound of the formula (I) and a sterile vehicle. The compound, depending on the vehicle and concentration used, can be either suspended or dissolved in the vehicle. In preparing solutions the compound can be dissolved for injection and filter sterilized before filling into a suitable vial or ampoule and sealing. Advantageously, adjuvants such as a local anaesthetic, preservatives and buffering agents can be dissolved in the vehicle. Parenteral

suspensions are prepared in substantially the same manner except that the compound is suspended in the vehicle instead of being dissolved and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

When appropriate, the compositions of this invention may be presented as an aerosol for oral administration, or as a microfine powder for insufflation.

As is common practice, the compositions will usually be accompanied by written or printed directions for use in the medical treatment concerned.

It will of course be realised that the precise dosage used in the treatment of any of the hereinbefore described disorders will depend on the actual compound of the formula (I) used, and also on other factors such as the seriousness of the disorder being treated.

The invention also provides a method of treatment and/or prophylaxis of disorders in human beings or domestic animals which comprises the administration to the sufferer of an effective amount of a compound of the formula (I).

The following Example illustrates the preparation of the active compounds of the invention.

Example 1

1-(9'-Hydroxydecyl)-2-(3"-hydroxy-3",4"-dimethyloctyl) -4-methyl-1,2,4-triazolidine-3,5-dione (Compound 1)

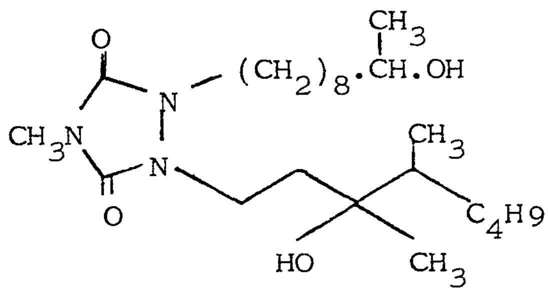

1-(9'-Oxodecyl)-2-(3"-hydroxy-3",4"-dimethyloctyl) -4-methyl-1,2,4-triazolidine-3,5-dione (375 mg; 0.88 mmol) in dry ethanol (15 ml) was stirred at room temperature and sodium borohydride (50 mg) added portionwise over 2 hr. After stirring for a further 30 min., the excess of borohydride was decomposed with glacial acetic acid and the mixture evaporated *in vacuo*. The residue was partitioned between water (50 ml) and ether (50 ml), and the organic layer washed with water (1 x 50 ml), brine (1 x 50 ml), dried ($Na_2SO_4$) and evaporated *in vacuo* gave 1-(9'-hydroxydecyl)-2-(3"-hydroxy-3",4"-dimethyl octyl)-4-methyl-1,2,4-triazolidine-3,5-dione as a colourless gum (368 mg; 97%).

Compounds 2 to 8 shown in Table 1 were prepared in a similar manner.

Table 1

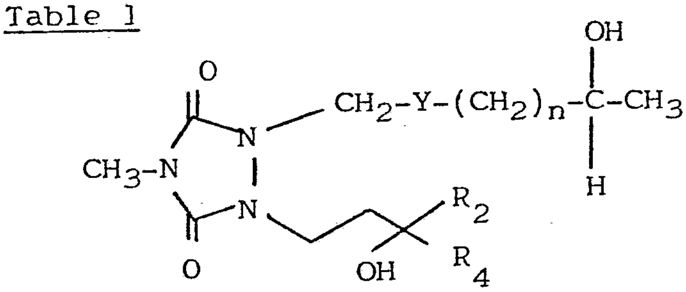

| Compound | n | Y | $R_2$ | $R_4$ |
|---|---|---|---|---|
| 1 | 5 | $-CH_2-CH_2-$ | $CH_3$ | $CH(CH_3)C_4H_9$ |
| 2 | 4 | $-CH_2-CH_2-$ | H | $C_5H_{11}$ |
| 3 | 4 | $-CH\overset{c}{=}CH-$ | $CH_3$ | $CH(CH_3)C_4H_9$ |
| 4 | 4 | $-CH_2-CH_2-$ | $CH_3$ | $C_6H_{13}$ |
| 5 | 3 | $-CH_2-CH_2-$ | $CH_3$ | $C_6H_{13}$ |
| 6 | 5 | $-C\equiv C-$ | $CH_3$ | $CH(CH_3)C_4H_9$ |
| 7 | 4 | $-CH\overset{c}{=}CH-$ | $CH_3$ | 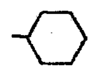 |
| 8 | 4 | $-C\equiv C-$ | $CH_3$ | 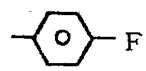 |

Analytical Data

Compound 1

I.R. ($cm^{-1}$): 3450 (O-H); 1765 and 1690 (N-C=O).

N.M.R. ($CDCl_3$) $\tau$ : 6.00-6.65 (7H, m, 2 x N-$\underline{CH_2}$ + 2 x OH + $\underline{CH}$-O protons);
6.95 (3H, s, N-$\underline{CH_3}$);
8.10- 9.20 (32H, m, $C_8H_{15}$ + $-(CH_2)_7-$ chains + $\underline{CH_3}$-);
8.88 (3H, s, $\underline{CH_3}$-tertiary alcohol).

Compound 2

I.R. ($cm^{-1}$): 3450 (O-H); 1765 and 1690 (N-C=O).

N.M.R. ($CDCl_3$) $\tau$ : 5.62 (2H, s, ex $D_2O$, 2 x OH);
6.00-6.70 (6H, m, 2 x N-$\underline{CH_2}$ and 2 x $\underline{CH}$-O protons);
6.95 (3H, s, N-$\underline{CH_3}$);
8.20-9.00 (22H, m, $-(CH_2)_6-$ and $-(CH_2)_5-$ chains);
8.84 (3H, d, J 6Hz, $\underline{CH_3}$.CH.O);
9.12 (3H, m, terminal $CH_3$-).

Compound 3

I.R. ($cm^{-1}$): 3450 (O-H); 1765 and 1700 (N-C=O); 1590 (C=C).

N.M.R. ($CDCl_3$) $\tau$ : 4.12-4.80 (2H, m, olefinic);
5.75 (2H, d, N-$\underline{CH_2}$.C=C);
6.00-6.50 (5H, m, N-$\underline{CH_2}$ + $\underline{CH}$.$\underline{OH}$ + OH);
6.94 (3H, s, N-$\underline{CH_3}$);
7.65-8.00 (2H, m, C=C.$\underline{CH_2}$);

Compound 4

I.R. (c.m.$^{-1}$) : 3450 (OH), 1690 and 1760 (N-C=O).

N.M.R. ($CDCl_3$)τ: 6.0-6.6 (5H, m, 2 x $NCH_2$, OH);
6.95 (3H, s, $NCH_3$);
8.0-8.9 (30H, m, $C_8H_{15}$ + $(CH_2)_6$ chain + $CH_3$)
9.1 (3H, brt, $(CH_2)_5\underline{CH_3}$).

Mass Spectrum: m/e ($M^*$) $C_{22}H_{43}N_3O_4$ requires 413.3254
found 413.3240

Compound 5

I.R. (cm$^{-1}$) : 3450 (OH), 1770 and 1690 (N-C=O)

N.M.R. ($CDCl_3$)τ: 6.0-6.6 (5H, m, 2 x $NCH_2$ + C$\underline{H}$-O)
6.93 (3H, s, N-$CH_3$)
7.85 (2H, s, 2 x OH)
8.10 - 9.15 (31H, m, $C\underline{H}_3$-CHO,
$(CH_2)_5$ and $C_9H_{18}$ chains)

Mass Spectrum: m/e (M*) $C_{21}H_{41}N_3O_4$ requires 399.3097
found 399.3086

Compound 6

N.M.R. ($CDCl_3$)τ: 5.73 (2H, t, N.$CH_2$.C≡C);
6.00 - 6.60 (3H, m, N.$CH_2$ + C$\underline{H}$OH);
6.95 (3H, s, N-$CH_3$);
7.20 (2H, s, ex $D_2O$; 2 x OH);
7.60 - 9.30 (31H, m, $C_9H_{18}$ and $C_6H_{13}$ chains)

Mass spectrum: m/e ($M^*$) $C_{23}H_{41}N_3O_4$ requires 423.3097
found 423.3102

Compound 7

I.R. (cm$^{-1}$) : 3430 (O-H); 2920 and 2840 (C-H); 1760 and 1680 (N-C=O).

N.M.R. ($CDCl_3$)τ: 4.10-4.90 (2H, m, olefinic protons);

5.60-6.65 (5H, m, 2 x N.$CH_2$ and CH,OH protons);
6.95 (3H, s, N-$CH_3$);
7.10 (2H, s, ex $D_2O$, 2 x OH)
7.60 - 9.30 (21H, m, -$(CH_2)_4$ -chain, + $CH_2$ + cyclohexyl);
8.81 (3H, d, J7.5 Hz, $CH_3$.CH.OH); 8.90 (3H, s, tertiary $CH_3$).

Mass spectrum: m/e (M*) $C_{22}H_{39}N_3O_4$ requires 409.2939
found 409.2925

Compound 8

I.R. (c.m.$^{-1}$) : 3450 (O-H); 2940 (C-H); 2220 (C≡C-)
1770 and 1695 (N-C=O); 1600 (aromatic C=C)

N.M.R. ($CDCl_3$)τ: 2.45-3.20 (4H, m, aromatic protons);
5.90 (2H, t, N.$CH_2$.C≡C);
6.20-6.70 (3H, m, N-$CH_2$ and CH.OH protons);
7.05 (3H, s, N-$CH_3$);
7.65 - 8.20 (4H, m, 2 x $CH_2$);
8.53 (3H, s, tertiary $CH_3$);
8.50-8.80 (6H, m, -$(CH_2)_3$-chain);
8.90(3H, d, J7.5 Hz, $CH_3$.CH.OH)

Mass Spectrum: m/e (M*) $C_{22}H_{30}N_3O_4F$ requires 419.2217
found 419.2227

## Pharmacological Data Section

### Bronchodilation Activity

The compounds were examined for their ability to inhibit 5-hydroxytryptamine or histamine induced bronchoconstriction in the anaesthetised artificially respired guinea-pig (Konzett-Rossler preparation).

| Compound | $ED_{50}$ (µg per kg i.v.) |
|---|---|
| 1 | 20.0 |
| 3 | 17.5 |
| 4 | 4.2 |
| 7 | 160 |

### Toxicity

No toxic effects were observed in this test.

Claims

1. A compound of the formula (I):

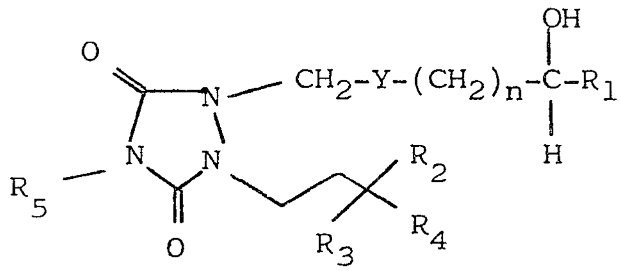

(I)

wherein:

n is 3 to 5;

Y is $-CH_2-CH_2$ or $-CH=CH-$ or $-C \equiv C-$

$R_1$ is $C_{1-4}$ alkyl;

$R_2$ is hydrogen, $C_{1-4}$ alkyl or phenyl;

$R_3$ is hydroxy or protected hydroxy;

$R_4$ is hydrogen, $C_{1-9}$ alkyl, $C_{3-8}$ cycloalkyl, phenyl, naphthyl, any of which phenyl moieties or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; and

$R_5$ is $C_{1-6}$ alkyl.

2. A compound according to claim 1 of formula (II):

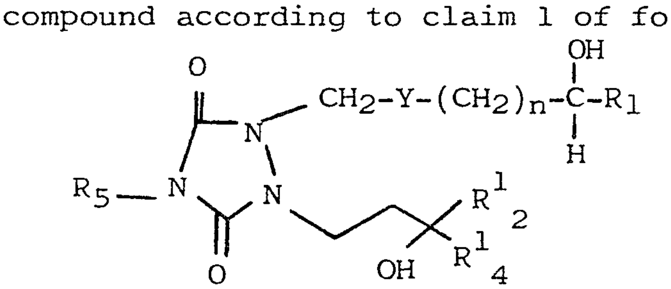

(II)

wherein:

Y, n, $R_1$ and $R_5$ are as defined in claim 1;

$R^1_2$ is hydrogen, methyl, ethyl or phenyl, and

$R^1_4$ is hydrogen or $C_{1-9}$ alkyl.

3. A compound according to claim 2 of formula (III):

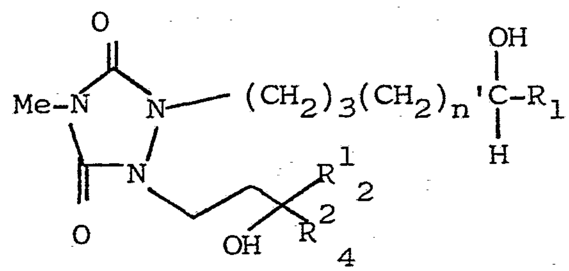

(III)

wherein:

n' is 4 or 5

$R_1$ is as defined in claim 1;

$R^1_2$ is hydrogen, methyl or ethyl; and

$R^2_4$ is a $C_{4-9}$ alkyl group.

4. 1-(8'-Hydroxy-n-nonyl)-2-(3"-hydroxy-3"-methyl-n-nonyl)-4-methyl-1,2,4-triazolidine-3,5-dione.

5. A compound according to claim 2 of formula (IV):

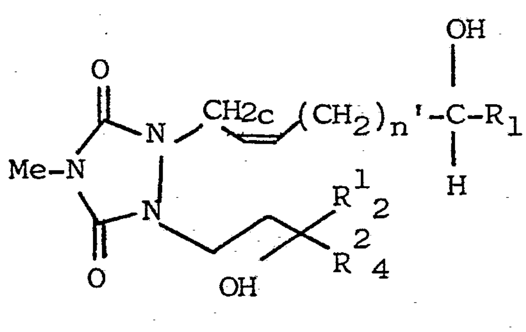

(IV)

wherein the variables are as defined in claim 3.

6. 1-(cis-8"-Hydroxy-non-2-enyl)-2-(3"-hydroxy-3",4"-dimethyloctyl)-4-methyl-1,2,4-triazolidine-3,5-dione.

7. A compound according to claim 1 of formula (VII):

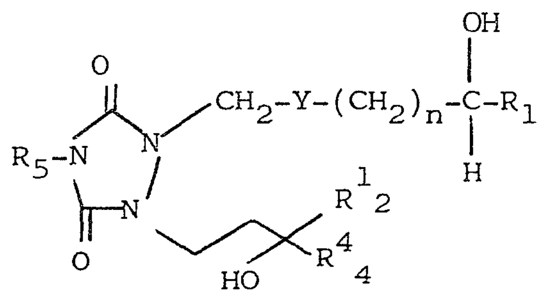

(VII)

wherein the variable groups are as defined in claim 2 and $R^4_4$ is a group of formula (VIII):

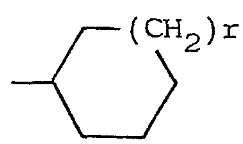

(VIII)

wherein r is 0-3.

8. A pharmaceutical composition comprising a compound according to claim 1 together with a pharmaceutically acceptable carrier.

9. A pharmaceutical composition comprising the compound of claim 4 or claim 6 together with a pharmaceutically acceptable carrier.

10. A process for the preparation of a compound according to claim 1 characterised by either

a) reacting a compound of formula (X):

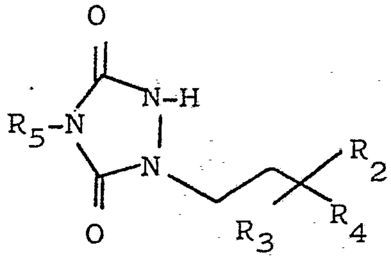

(X)

wherein $R_2$, $R_3$, $R_4$, and $R_5$ are as defined in claim 1 with a compound of formula (XI):

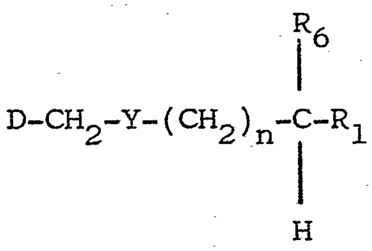

(XI)

wherein D is a group readily displaced by nucleophiles; $R_6$ is hydroxy or protected hydroxy; and Y, n and $R_1$ are as defined in claim 1, and thereafter if necessary deprotecting the $R_6$ hydroxy moiety;

or b) reacting a compound of formula (XII):

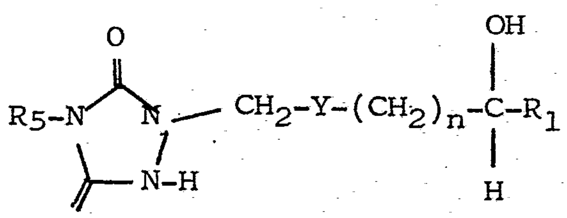

wherein:

Y is $CH_2$-$CH_2$- or -CH=CH-; and n, $R_1$ and $R_5$ are as defined in claim 1 with a compound of formula (XIII):

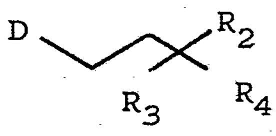

(XIII)

wherein D is a group readily displaced by necleophiles, and $R_2$, $R_3$ and $R_4$ are as defined in claim 1;

or c) reacting a compound of the formula (XIV):

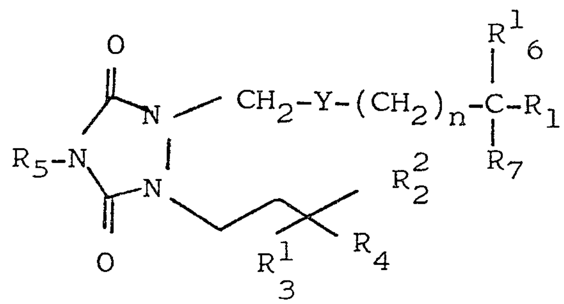

(XIV)

wherein $R^2_2$ and $R^1_3$ are as defined for $R_2$ and $R_3$ in formula (I) or $R^2_2$ and $R^1_3$ together form an oxo group; $R^1_6$ is hydroxy or protected hydroxy; $R_7$ is hydrogen or $R^1_6$ and $R_7$ together form an oxo group and at least one of $R^2_2$ + $R^1_3$ and $R^1_6$ + $R_7$ are oxo with either i) a carbonyl reducing agent or (when $R^1_6$ is hydroxy or protected hydroxy) ii) an alkyl or phenyl Grignard reagent (or similar metallic complex) and thereafter deprotecting $R^1_6$ when protected hydroxy.

Claims

1. A process for the preparation of a compound of the formula (I):

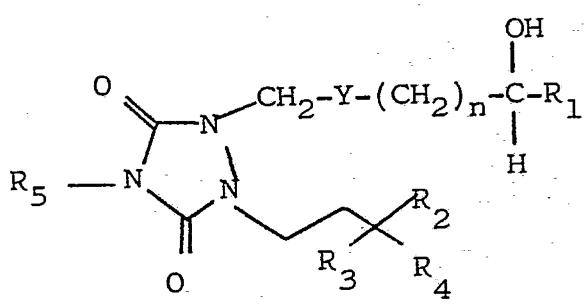

(I)

wherein:
n is 3 to 5;
Y is $-CH_2-CH_2$ or -CH=C-
$R_1$ is $C_{1-4}$ alkyl;
$R_2$ is hydrogen, $C_{1-4}$ alkyl or phenyl;
$R_3$ is hydroxy or protected hydroxy;
$R_4$ is hydrogen, $C_{1-9}$ alkyl, $C_{3-8}$ cycloalkyl, phenyl, naphthyl, any of which phenyl moieties or naphthyl moieties may be substituted by one or more halogen, trifluoromethyl, $C_{1-6}$ alkyl, hydroxy, $C_{1-6}$ alkoxy, phenyl $C_{1-6}$ alkoxy or nitro groups; and
$R_5$ is $C_{1-6}$ alkyl
characterised by either
a) reacting a compound of formula (X):

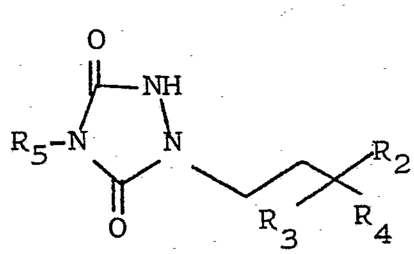

(X)

wherein $R_2$, $R_3$, $R_4$, and $R_5$ are as herinbefore defined with a compound of formula (XI):

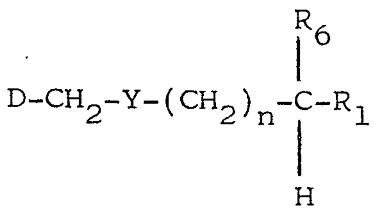

(XI)

wherein D is a group readily displaced by nucleophiles; $R_6$ is hydroxy or protected hydroxy; and Y, n and $R_1$ are as hereinbefore defined, and thereafter if necessary deprotecting the $R_6$ hydroxy moiety;

or b) reacting a compound of formula (XII):

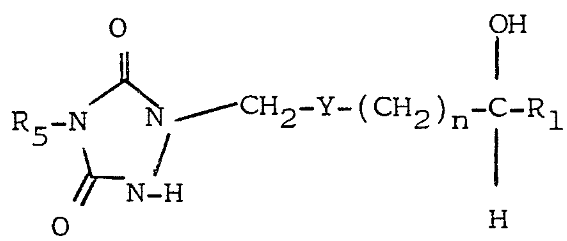

(XII)

wherein:

Y is $CH_2$-$CH_2$- or -CH=CH-; and

n, $R_1$ and $R_5$ are as hereinbefore defined with a compound of formula (XIII):

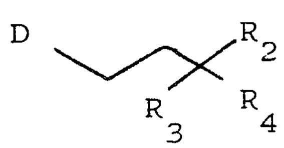

(XIII)

wherein D is a group readily displaced by nucleophiles, and $R_2$, $R_3$ and $R_4$ are as hereinbefore defined;

or c) reacting a compound of the formula (XIV):

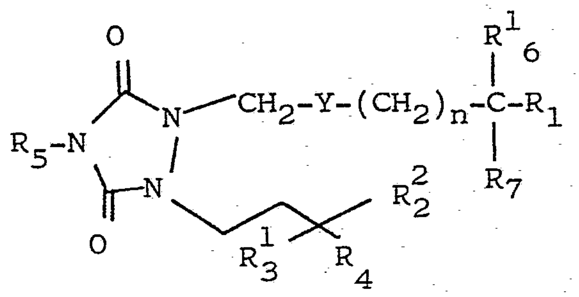

(XIV)

wherein $R^2_2$ and $R^1_3$ are as defined for $R_2$ and $R_3$ in formula (I) or $R_2^2$ and $R^1_3$ together form an oxo group; $R^1_6$ is hydroxy or protected hydroxy; $R_7$ is hydrogen or $R^1_6$ and $R_7$ together form an oxo group and at least one of $R^2_2 + R^1_3$ and $R^1_6 + R_7$ are oxo with either i) a carbonyl reducing agent or (when $R^1_6$ is hydroxy or protected hydroxy) ii) an alkyl or phenyl Grignard reagent (or similar metallic complex) and thereafter deprotecting $R^1_6$ when protected hydroxy.

2. A process according to claim 1 for the preparation of a compound of formula (II):

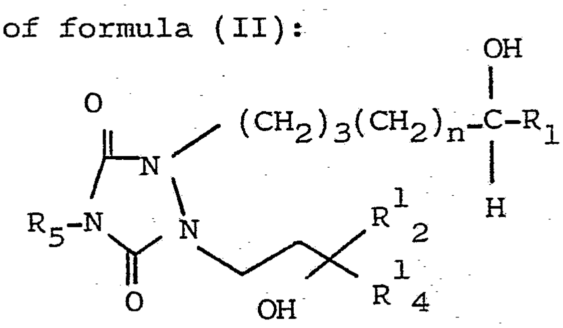

(II)

wherein:

Y, n, $R_1$ and $R_5$ are as defined in claim 1;
$R^1_2$ is hydrogen, methyl, ethyl or phenyl; and
$R^1_4$ is hydrogen or $C_{1-9}$ alkyl.

3. A process according to claim 2 for the preparation of a compound of formula (III):

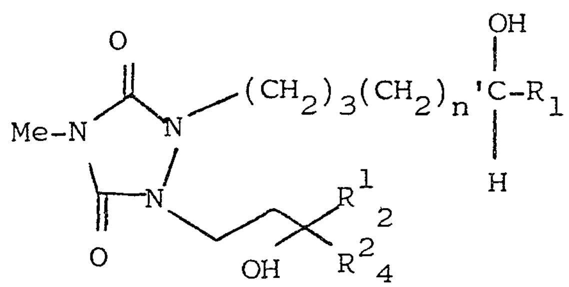

(III)

wherein:

n' is 4 or 5,

$R_1$ is as defined in claim 1;

$R^1_2$ is hydrogen, methyl or ethyl; and

$R^2_4$ is a $C_{4-9}$ alkyl group.

4. A process according to claim 3 for the preparation of 1-(8'-Hydroxy-n-nonyl)-2-(3"-hydroxy-3"-methyl-n-nonyl)-4-methyl-1,2,4-triazolidine-3,5-dione characterised by either:

a) reacting the compound of formula (X) as defined in claim 1 wherein $R_2$ is $CH_3$, $R_3$ is hydroxy, $R_4$ is n $-C_6H_{13}$ and $R_5$ is $CH_3$ with the compound of formula (XI) as defined in claim 1 wherein n is 4, Y is $-CH_2-CH_2-$, $R_1$ is $CH_3$ and $R_6$ is protected hydroxy and thereafter deprotecting the $R_6$ hydroxy moiety;

or b) reacting the compound of formula (XII) as defined in claim 1 wherein Y, n, $R_1$ and $R_5$ are as hereinbefore defined with the compound of formula (XIII) as defined in claim 1 wherein $R_2$, $R_3$ and $R_4$ are as hereinbefore defined;

or c) reacting the compound of formula (XIV) as defined in claim 1 wherein Y, n, $R_1$, $R_2$, $R_3$, $R_4$ and $R_5$ are as hereinbefore defined with a carbonyl reducing agent or methyl Grignard reagent (or methyl metallic complex), as appropriate.

5. A process according to claim 2 for the preparation of a compound of the formula (IV):

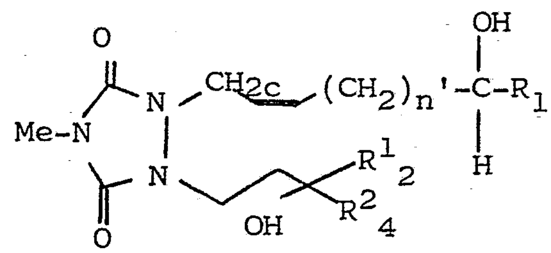

(IV)

wherein the variables are as defined in claim 3.

6. A process according to claim 5 for the preparation of 1-(<u>cis</u>-8"-Hydroxy-non-2-enyl)-2-(3"-hydroxy-3",4"-dimethyloctyl)-4-methyl-1,2,4-triazolidine-3,5-dione characterised by either:
a) reacting the compound of formula (X) as defined in claim 1 wherein $R_2$ is $CH_3$, $R_3$ is hydroxy, $R_4$ is $CH(CH)C_4H_9$ and $R_5$ is $CH_3$ with the compound of formula (XI) as defined in claim 1 wherein n is 4, Y is -CH=CH-, R is $CH_3$ and $R_6$ is protected hydroxy and thereafter deprotecting the $R_6$ hydroxy moiety;
or b) reacting the compound of formula (XII) as defined in claim 1 wherein Y, n, $R_1$ and $R_5$ are as hereinbefore defined with the compound of formula (XIII) as defined in claim 1 wherein $R_2$, $R_3$ and $R_4$ are as hereinbefore defined;
or c) reacting the compound of formula (XIV) as defined in claim 1 wherein Y, n, $R_1$, $R_2$, $R_3$, $R_4$ and $R_5$ are as hereinbefore defined, with a carbonyl reducing agent or methyl Grignared reagent (or methyl metallic complex), as appropriate.

7. A process according to claim 1 for the preparation of a compound of formula (VII):

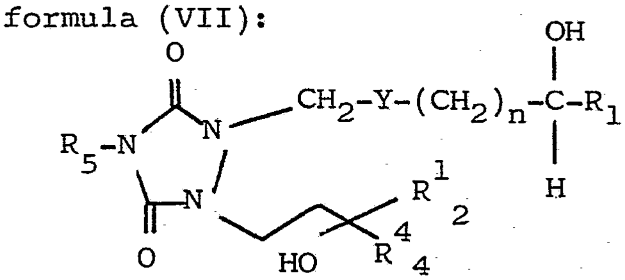

(VII)

wherein the variables groups are as defined in claim 2 and $R^4{}_4$ is a group of formula (VIII):

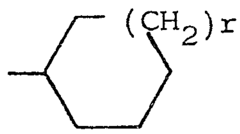

(VIII)

wherein r is 0-3.

0024105

European Patent Office

# EUROPEAN SEARCH REPORT

Application number
EP 80 30 2206

## DOCUMENTS CONSIDERED TO BE RELEVANT

| Category | Citation of document with indication, where appropriate, of relevant passages | Relevant to claim |
|---|---|---|
| P/A | EP - A - 0 007 180 (BEECHAM)<br>* Claims *<br>----- | 1 |

CLASSIFICATION OF THE APPLICATION (Int Cl.³)

C 07 D 249/12
A 61 K 31/41

TECHNICAL FIELDS SEARCHED (Int. Cl.³)

C 07 D 249/12
A 61 K 31/41

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 20-10-1980 | BERTE |